# EUROPEAN PATENT SPECIFICATION

(11) **EP 4 297 860 B1**
(45) Date of publication and mention of the grant of the patent: **09.04.2025**
(21) Application number: 22701302.6
(22) Date of filing: 11.01.2022
(51) Int. Cl.: A61N 1/37, A61N 1/08

(54) **IMPLANTABLE MEDICAL DEVICE EMPLOYING AN AUTOMATIC ADJUSTMENT OF SENSING AND STIMULATION PARAMETERS UPON SWITCHING FROM A BIPOLAR OPERATIONAL MODE TO A UNIPOLAR OPERATIONAL MODE**
IMPLANTIERBARE MEDIZINISCHE VORRICHTUNG MIT AUTOMATISCHER ANPASSUNG DER ABTAST- UND STIMULATIONSPARAMETER BEIM UMSCHALTEN VON EINEM BIPOLAREN BETRIEBSMODUS IN EINEN UNIPOLAREN BETRIEBSMODUS
DISPOSITIF MÉDICAL IMPLANTABLE UTILISANT UN RÉGLAGE AUTOMATIQUE DES PARAMÈTRES DE DÉTECTION ET DE STIMULATION LORS DU PASSAGE D'UN MODE OPÉRATIONNEL BIPOLAIRE À UN MODE OPÉRATIONNEL UNIPOLAIRE

(30) Priority: 26.02.2021 EP 21159500
(43) Date of publication of application: 03.01.2024
(73) Proprietor: BIOTRONIK SE & Co. KG, 12359 Berlin (DE)
(72) Inventor: BECKER, Frank, 10409 Berlin (DE)
(74) Representative: Biotronik Corporate Services SE
(86) International application number: PCT/EP2022/050371
(87) International publication number: WO 2022/179762

(56) References cited:
- US-B1- 6 546 288
- US-B1- 7 308 310
- US-B1- 7 574 259

## Description

The present invention relates to an implantable medical device for stimulating a human or animal heart according to claim 1, to an arrangement comprising such implantable medical device according to claim 9, and to a computer program product according to claim 12.

Implantable medical devices for stimulating a human or animal heart, such as pacemakers, have been known for a long time. They can perform different functions. Different stimulation programs can be carried out by an appropriate pacemaker to restore the treated heart to a normal state. Pacemakers are also known to stimulate the His bundle.

The His bundle is a bundle of specific heart muscle cells that is part of the cardiac conduction system. The His bundle is located distally of the atrioventricular node towards the apex of the heart.

There exist specific devices adapted for His bundle pacing, wherein a detecting (sensing) and stimulation electrode is not implanted into the ventricle of the human or animal heart to be treated, but rather at or near to the His bundle of the heart. Such use of a His bundle electrode enables a particularly physiologic stimulation of the human or animal heart.

It is generally known that an operational mode of an implantable medical device for stimulating a human or animal heart can be switched from a bipolar sensing/stimulation to a unipolar sensing/stimulation if an electrode failure has occurred that prevents from operating the implantable medical device safely in a bipolar manner. This switching is typically done without adjusting the sensing/stimulation parameters. While such an approach is feasible (although not optimal) in case of a right ventricular stimulation, it can have severe medical side-effects in case of a His bundle stimulation since the His bundle is much more sensitive to delivered electric stimulation pulses than the right ventricle is.

US 7,308,310 Bl discloses an implantable cardiac stimulation device which provides bipolar pacing and bipolar autocapture. If a measured bipolar electrode configuration capture threshold is too high, an impedance measuring circuit measures the impedance of the bipolar electrode configuration to determine a possible lead failure. If the bipolar electrode configuration impedance is within a normal range, bipolar pacing is continued in a manner which assures continued capture of the heart. If the bipolar electrode configuration impedance is outside of the given range of impedances, the device is switched to unipolar pacing in a manner which insures capture of the heart and notification of the need for a physician consultation.

US 7,574,259 Bl discloses a method which includes performing a capture threshold assessment using a bipolar electrode configuration, deciding if capture occurred for a maximum energy value of the capture threshold assessment and, if capture did not occur, then performing a lead impedance test for the lead associated with the bipolar electrode configuration. Such a test may aim to detect an insulation defect and/or a conductor defect. Other exemplary methods, devices, systems, etc., are also disclosed.

US 6,546,288 Bl discloses an implantable cardiac stimulation device and associated method which provide automatic capture verification and threshold testing capabilities with an added high-threshold response algorithm and patient notification process. The stimulation device responds to higher than normal capture thresholds by permitting the stimulation pulse energy to be set above the fixed maximum pulse energy normally allowed by automatic capture verification techniques. In order to maintain comfortable yet effective stimulation, the high-energy stimulation is delivered in a bipolar configuration. The stimulation device alerts the patient of the change to high-energy output when it occurs. After the high-energy output is automatically set, one or more high-energy stimulation pulses are delivered in a unipolar configuration on a scheduled or event triggered basis. A unipolar high-energy stimulation pulse is typically perceptible by the patient due to stimulation of excitable tissue surrounding the device housing. Sensation of a periodic high-energy pulse alerts the patient that stimulation conditions have changed and medical attention should be sought.

It is an object of the present invention to provide an implantable medical device for stimulating a cardiac region in a safe and reliable way irrespective of the implantable medical device being operated in a unipolar manner or in a bipolar manner.

This object is achieved with an implantable medical device for stimulating a human or animal heart having the features of claim 1. Such an implantable medical device comprises a housing, a processor, a memory unit, a stimulation unit, and a detection unit. The stimulation unit is configured to stimulate a cardiac region of a human or animal heart. The detection unit is configured to detect an electrical signal at the cardiac region of the same heart. Furthermore, the stimulation unit comprises an electrode having a first electrode pole and a second electrode pole. Thus, the electrode of the stimulation unit is arranged and designed to stimulate the cardiac region in a bipolar manner.

The memory unit comprises a computer-readable program that causes the processor to perform the steps explained in the following when executed on the processor.

**In** one of the steps, appropriate stimulation parameter values of a stimulation pulse are determined. The stimulation pulse is to be delivered to a cardiac region of a human or animal heart by the first electrode pole in a unipolar manner. Typically, these stimulation parameter values differ from stimulation parameter values of a stimulation pulse to be delivered by the same electrode, but in a bipolar manner, i.e., by using the first electrode pole and the second electrode pole.

In another of the steps, the determined stimulation parameter values are stored in the memory unit of the implantable medical device.

In another of the steps, an occurrence of an electrode failure (electrode error) of the electrode is checked. This check takes place as long as the electrode is operated in a bipolar manner.

In this context, it is of no importance in which order the precedingly explained steps are performed. Thus, it is possible to determine appropriate stimulation parameter values of the unipolar stimulation pulse and subsequently storing them in the memory unit prior to or after checking the occurrence of an electrode failure. In an embodiment, the steps are performed in the order as indicated in the claims. Then, appropriate stimulation parameter values are already stored in the memory unit of the implantable medical device once an electrode failure test is performed.

In case that an electrode failure has been detected, the implantable medical device automatically switches from a bipolar operation of the electrode to a unipolar operation of the electrode. In doing so, the stored stimulation parameter values for unipolar stimulation of the cardiac region are automatically applied by the device. Thus, the stimulation unit is prepared to deliver stimulation pulses having the stored stimulation parameter values if the implantable medical device has been switched to operate in a unipolar manner.

Thus, the presently claimed implantable medical device does not only provide for an automatic switch from a bipolar operation to a unipolar operation in case of a detected electrode failure, but also for an automatic adaptation of the stimulation parameter values to be used for subsequent stimulations in a unipolar manner. These stimulation parameter values of a unipolar stimulation are specifically adapted to such a unipolar stimulation. Therefore, a subsequent unipolar stimulation results in a safe and reliable stimulation of the cardiac region to be stimulated as well as avoiding severe side-effects of a non-physiologic stimulation. Consequently, these stimulation parameter values for a unipolar stimulation are much better suited for such unipolar stimulation than stimulation parameter values adapted for bipolar stimulation that are typically used by the implantable medical device when applying a bipolar stimulation of the cardiac region.

In an embodiment, the implantable medical device is an implantable pulse generator (IPG), an implantable cardioverter-defibrillator (ICD), or a device for cardiac resynchronization therapy (CRT).

Appropriate stimulation parameter values for a unipolar stimulation can be, e.g., obtained when performing a threshold test to determine appropriate stimulation value parameters for a bipolar stimulation. Such a threshold test needs to be done at least once prior to a first stimulation in order to obtain information on the stimulation threshold. This threshold depends on physiological parameters of the heart to be stimulated as well as on necessary compensations of implantation-specific conductance variances. In many cases, such threshold test is regularly done, e.g., once a day, once every two days, once a week, once a month, once a year, or in any time period lying in an interval that can be built up from the precedingly mentioned time points (e.g., between once a day and once a year).

In an embodiment, the implantable medical device does not only serve for an automatic adaptation of stimulation parameter values, but also of an automatic adaptation of sensing parameter values. In this embodiment, the detection unit comprises the same electrode as the stimulation unit. Furthermore, the computer-readable program causes the processor to determine appropriate sensing parameter values for sensing an electric signal at a cardiac region by the first electrode pole in a unipolar manner and to store the determined sensing parameter values in the memory unit prior to automatically switching from a bipolar operation of the electrode to a unipolar operation of the electrode. Furthermore, the computer-readable program causes the processor to automatically apply the stored sensing parameter values for sensing an electrical signal at a cardiac region upon switching from a bipolar operation of the electrode to a unipolar operation of the electrode. In this context, the appropriate sensing parameter values can be determined prior to or after determining the appropriate stimulation parameter values. It would also be generally possible to determine one of the appropriate stimulation parameter values and the appropriate sensing parameter values prior to checking the occurrence of an electrode failure and to determine the respective other of the sensing parameter values and the stimulation parameter values after checking the occurrence of an electrode failure. However, typically, both the appropriate stimulation parameter values and the appropriate sensing parameter values will be determined prior to checking for electrode failure. Then, the corresponding values are already stored in the memory unit and can be immediately used for subsequent stimulation and/or sensing events without temporal delay.

Appropriate sensing parameter values for a unipolar sensing can be typically obtained upon determining sensing parameter values for a bipolar sensing. In many cases, such determining is regularly done, e.g., once a day, once every two days, once a week, once a month, once a year, or in any time period lying in an interval that can be built up from the precedingly mentioned time points (e.g., between once a day and once a year).

In an embodiment, the stimulation parameter comprises an amplitude and/or a pulse width of a stimulation pulse to be subsequently delivered. Expressed in other words, the stimulation parameter value comprises a value of an amplitude and/or a value of a pulse width of a stimulation pulse to be delivered at a later time.

In an embodiment, the value of the amplitude is a value chosen from a range of from 0.2 V to 10 V, in particular from 0.2 V to 7.5 V, in particular from 0.4 V to 7.0 V, in particular from 0.8 V to 6.0 V, in particular from 1 V to 5 V,.

In an embodiment, the value of a pulse width is a value chosen from a range of from 0.1 ms to 5 ms, in particular from 0.2 ms to 3 ms, in particular from 0.4 ms to 1.5 ms,

In an embodiment, the cardiac region is a right ventricle of the human or animal heart. Thus, in this embodiment, the implantable medical device is a conventional-type device that is able to stimulate the right ventricle of the human or animal heart. While the ventricular tissue is relatively tolerant with respect to variations of stimulation parameter values, an adaptation of the stimulation parameter values to a unipolar stimulation in case of necessary unipolar operation of the implantable medical device results in a more physiologic stimulation of the right ventricular tissue.

In an embodiment, the cardiac region is a His bundle of the human or animal heart. The His bundle stimulation is much more critical with respect to the applied stimulation parameter values and/or to the sensing parameter values than the right ventricle is. A unipolar stimulation of the His bundle with a parameter set adapted for a bipolar stimulation might either result in an over- stimulation or in an under-stimulation. If no sufficient stimulation of the His bundle is achieved, no cardiac contraction will be evoked by this under-stimulation. Then, the implantable medical device does not achieve its intended purpose. If, however, an over-stimulation takes place, the stimulation is not optimal for the patient which leads to a reduction in everyday life quality

In an embodiment, the steps of determining appropriate stimulation parameter values, storing the determined stimulation parameter values and checking an occurrence of an electrode failure are carried out automatically or upon a user's request. In this context, it is possible that one or more of the steps are carried out automatically, wherein one or more other of the steps are carried out upon a user's request. There is no dependency between the individual steps in this respect. To give an example, an operating interface can offer to determine appropriate stimulation parameter values. If a user (such as a physician) accepts this offer, e.g., by clicking on a corresponding button, the step of determining the stimulation parameter values will be performed by the device.

If the steps of determining appropriate sensing parameter values and storing these sensing parameter values are carried out, the steps can also be performed automatically or upon a user's request. Once again, there is no interdependency between individual ones of these steps or between these steps and the precedingly explained steps relating to the determining and storing the stimulation parameter values or to the electrode failure test.

In an embodiment, the step of checking the occurrence of an electrode failure is carried out automatically. In an embodiment, this step is cyclically repeated. For this purpose, it is possible to regularly check the occurrence of an electrode failure in predefined time intervals, such as once a heart cycle, a minute, a hour, a day, once a week, once a month, once a quarter or once a year. Furthermore, it is possible to perform such a regular electrode failure check in a frequency falling within a time interval that can be built up from the precedingly mentioned time points (e.g., between once a day and once a year).

In an embodiment, the step of checking the occurrence of an electrode failure is carried out by measuring an impedance between the first electrode pole and the second electrode pole. This impedance is then compared with a predetermined lower impedance threshold value and a predetermined upper impedance threshold value. If the measured impedance is lower than the lower impedance threshold value or higher than the upper impedance threshold value, this is taken as an indication that an electrode failure has occurred. In such a case, the implantable medical device automatically switches from the current bipolar operational mode to a unipolar operational mode. Thus, subsequent sensing and/or stimulation events will only be carried out in a unipolar manner. For such subsequent sensing and/or stimulation events, only the previously stored stimulation parameter values and/or sensing parameter values will be chosen.

In an embodiment, the electrode failure is an electrode kink or an electrode breakage. In an embodiment, the electrode failure only affects an electric path between the first electrode pole and the second electrode pole.

In an aspect, the present invention relates to an arrangement comprising an implantable medical device according to the preceding explanations and a programming device for programming the implantable medical device. With such a programming device, individual parameters of the implantable medical device can be set. Furthermore, specific measurements or tests can be initiated with the help of such programming device.

The programming device can, e.g., enable the possibility to start or repeat individual measurements or determinations of specific parameters, such as stimulation parameter values and/or sensing parameter values being appropriate for a unipolar stimulation/sensing by the electrode.

In an embodiment, the programming device is configured to enable an adjustment of stimulation parameter values of the stimulation pulse to be delivered to a cardiac region of a human or animal heart by the first electrode pole of an electrode in a unipolar manner. Alternatively or additionally, the programming device is configured to enable storing of the stimulation parameter values. Thus, the programming device can be used to adjust certain stimulation parameter values that have been previously determined by the implantable medical device. Such an adjustment offers higher flexibility of the method performed by the implantable medical device.

In an embodiment, the programming device comprises a memory and a processor, wherein the memory comprises a computer-readable program that causes the processor to perform the step of enabling an adjustment of stimulation parameter values and/or of enabling storing of the (adjusted) stimulation parameter values when executed on the processor.

In an embodiment, the programming device is configured to enable an adjustment of sensing parameter values for sensing an electrical signal at the cardiac region by the first electrode pole in a unipolar manner. Alternatively or additionally, the programming device is configured to enable storing of the (adjusted) sensing parameter values.

In an embodiment, the programming device comprises a memory and a processor, wherein the memory comprises a computer-readable program that causes the processor to perform the step of enabling an adjustment of sensing parameter values and/or of enabling storing of the (adjusted) sensing parameter values when executed on the processor.

In an embodiment, the implantable medical device comprises a data communication unit. In an embodiment, the data communication unit serves for transferring data to the programming device in a wireless manner. For such purpose, the programming device typically also comprises a data communication unit. All standard data transmission protocols or specifications are appropriate for such a wireless data communication. Examples of standard data transmission protocols or specifications are the Medical Device Radiocommunications Service (MICS), the Bluetooth Low Energy (BLE) protocol and the Zigbee specification.

In an aspect, the present invention relates to computer program product comprising computer-readable code that causes a device to execute the steps explained in the following.

Such device may be an implantable medical device comprising a housing, a processor, a memory unit, a stimulation unit, and a detection unit. The stimulation unit is configured to stimulate a cardiac region of a human or animal heart. The detection unit is configured to detect an electrical signal at the cardiac region of the same heart. Furthermore, the stimulation unit comprises an electrode having a first electrode pole and a second electrode pole. Thus, the electrode of the stimulation unit is arranged and designed to stimulate the cardiac region in a bipolar manner.

In one of the steps, appropriate stimulation parameter values of a stimulation pulse are determined. The stimulation pulse is to be delivered to a cardiac region of a human or animal heart in a unipolar manner by a first electrode pole of an electrode of a stimulation unit of the implantable medical device.

In another of the steps, the determined stimulation parameter values are stored in a memory unit of the implantable medical device.

In another of the steps, an occurrence of an electrode failure (electrode error) of the electrode is checked. This check takes place as long as the electrode is operated in a bipolar manner.

As explained above, it is of no importance in which order the precedingly explained steps are performed.

In case that an electrode failure has been detected, the implantable medical device automatically switches from a bipolar operation of the electrode to a unipolar operation of the electrode. In doing so, the stored stimulation parameter values for unipolar stimulation of the cardiac region are automatically applied by the device. Thus, the stimulation unit is prepared to deliver stimulation pulses having the stored stimulation parameter values if the implantable medical device has been switched to operate in a unipolar manner.

All embodiments of the implantable medical device can be combined in any desired way and can be transferred either individually or in any arbitrary combination to the described arrangement, to the described methods and the described computer program product. Furthermore, all embodiments described with respect to the arrangement can be combined in any desired way and can be transferred either individually or in any arbitrary combination to the described implantable medical device, to the described methods, or to the described computer program product. Likewise, all embodiments of the described methods can be combined in any desired way and can be transferred either individually or in any arbitrary combination to the respective other method, to the implantable medical device, to the arrangement, and to the computer program product. Finally, all embodiments described with respect to the computer program product can be combined in any desired way and can be transferred either individually or in any arbitrary combination to the described implantable medical device, to the described arrangement, or to the described methods.

Further details of aspects of the present invention will be described in the following exemplary embodiments and an accompanying Figure.

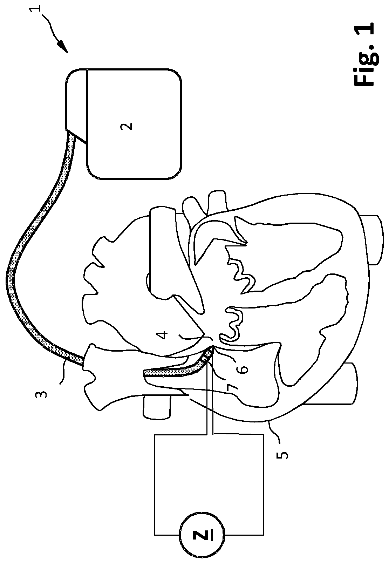

- Figure 1: shows a pacemaker employing an embodiment of a method for automatically switching the operational mode of the pacemaker.

Figure 1 shows a pacemaker 1 as implantable medical device. The pacemaker 1 comprises a housing 2. Furthermore, a His bundle electrode 3 is connected to the pacemaker 1 so as to be able to provide stimulation pulses to a His bundle 4 of a human heart 5 and to sense electric signals at the His bundle 4 of the human heart 5. For this purpose, the electrode 3 comprises at its distal tip a first electrode pole 6 and somewhat more proximally located a second electrode pole 7. The first electrode pole 6 is implanted at or near the His bundle 4 of the human heart 5. The second electrode pole 7 is configured as ring electrode pole.

The normal operational mode of the His bundle electrode 3 is a bipolar operational mode, i.e., the His electrode 3 employs a bipolar stimulation of the His bundle 4 and senses electric signals at the His bundle 4 in a bipolar manner. For this purpose, the first electrode pole 6 and the second electrode pole 7 are used.

During extended operation of the His bundle electrode 3, it is possible that an electrode failure occurs. Such an electrode failure can, e.g., be any error in the electric path between the first electrode pole 6 and the second electrode pole 7 which is used for conducting a bipolar sensing or a bipolar stimulation. In order to detect such electrode failure, an impedance Z is measured regularly between the first electrode pole 6 and the second electrode pole 7 in predetermined time intervals. If the measured impedance exceeds an upper impedance threshold of falls below a lower impedance threshold, the His bundle electrode 3 is considered to have an electrode failure (such as an electrode kink or an electrode breakage). In such a case, the implantable medical device 1 automatically switches from the previously applied bipolar operational mode to a unipolar operational mode. In such unipolar operational mode, a stimulation pulse will only be delivered with the first electrode pole 6, wherein the housing 2 of the pacemaker 1 is used as counter electrode.

In order to achieve a proper stimulation of the His bundle 4 also in case of a unipolar stimulation as well as to safely detect electric pulses at the His bundle 4 also in case of a unipolar sensing of electric signals, the pacemaker 1 applies a set of specific parameters adapted for such unipolar stimulation/sensing. For this purpose, appropriate stimulation parameter values and appropriate sensing parameter values have already been determined by the pacemaker 1 during the bipolar operation of the His bundle electrode 3. The determined values have been stored in a memory unit of the pacemaker 1 so that they can now be read from the memory and applied in subsequent stimulation pulses or measuring events that are carried out in a unipolar manner. Consequently, the pacemaker 1 continues to reliably work even in case of a failure of the His bundle electrode 3 by switching from a bipolar operational mode to a unipolar operational mode and by applying stimulation parameter values and/or sensing parameter values specifically adapted to such unipolar stimulation/sensing.

## Claims

1. Implantable medical device for stimulating a human or animal heart, comprising a housing (2), a processor, a memory unit, a stimulation unit configured to stimulate a cardiac region of a human or animal heart (5), and a detection unit configured to detect an electrical signal at the cardiac region of the same heart (5), wherein the stimulation unit comprises an electrode (3) having a first electrode pole (6) and a second electrode pole (7), wherein the memory unit comprises a computer-readable program that causes the processor to perform the following steps when executed on the processor:
a) determining appropriate stimulation parameter values of a stimulation pulse to be delivered to a cardiac region of a human or animal heart (5) by the first electrode pole (6) in a unipolar manner;
b) storing the determined stimulation parameter values in the memory unit;
c) checking an occurrence of an electrode failure of the electrode (3) when operating the electrode (3) in a bipolar manner;
d) in case of the occurrence of an electrode failure, automatically switching from a bipolar operation of the electrode (3) to a unipolar operation of the electrode (3) and automatically applying the stored stimulation parameter values for a unipolar stimulation of the cardiac region.

2. Implantable medical device according to claim 1, **characterized in that** the detection unit comprises the same electrode (3) as the stimulation unit, wherein the computer-readable program causes the processor to perform the following steps prior to step d): determining appropriate sensing parameter values for sensing an electrical signal at the cardiac region by the first electrode pole (6) in a unipolar manner and storing the determined sensing parameter values in the memory unit, wherein the computer-readable program causes the processor to perform additionally the following in step d): applying the stored sensing parameter values for sensing an electrical signal at the cardiac region.

3. Implantable medical device according to claim 1 or 2, **characterized in that** the stimulation parameter comprise a value of an amplitude and/or a value of a pulse width of a stimulation pulse.

4. Implantable medical device according to any of claims 1 to 3, **characterized in that** the cardiac region is a right ventricle of the human or animal heart (5).

5. Implantable medical device according to any of claims 1 to 3, **characterized in that** the cardiac region is a His bundle (4) of the human or animal heart (5).

6. Implantable medical device according to any of the preceding claims, **characterized in that** steps a), b), c) are, independently from each other, carried out automatically or upon a user's request.

7. Implantable medical device according to any of the preceding claims, **characterized in that** step c) is cyclically repeated.

8. Implantable medical device according to any of the preceding claims, **characterized in that** step c) is carried out by measuring an impedance (Z) between the first electrode pole (6) and the second electrode pole (7) and by determining whether the measured impedance (Z) is lower than a predetermined lower impedance threshold value or higher than a predetermined upper impedance threshold value.

9. Arrangement comprising an implantable medical device according to any of the preceding claims and a programming device for programming the implantable medical device (1).

10. Arrangement according to claim 9, **characterized in that** the programming device is designed and arranged to enable an adjustment of stimulation parameter values of a stimulation pulse to be delivered to a cardiac region of a human or animal heart (5) by a first electrode pole (6) of an electrode (3) in a unipolar manner and/or to enable storing of the stimulation parameter values.

11. Arrangement according to claim 9 or 10, **characterized in that** the programming device is designed and arranged to enable an adjustment of sensing parameter values for sensing an electrical signal at the cardiac region of a human or animal heart (5) by a first electrode pole (6) of an electrode (3) in a unipolar manner and/or to enable storing of the sensing parameter values.

12. Computer program product comprising computer-readable code that causes the device of claim 1 to execute the following steps:
a) determining appropriate stimulation parameter values of a stimulation pulse to be delivered to a cardiac region of a human or animal heart (5) by a first electrode pole (6) of an electrode (3) of a stimulation unit of an implantable medical device (1) in a unipolar manner;
b) storing the determined stimulation parameter values in a memory unit of the implantable medical device (1);
c) checking an occurrence of an electrode failure of the electrode (3) when operating the electrode (3) in a bipolar manner;
d) in case of the occurrence of an electrode failure, automatically switching from a bipolar operation of the electrode (3) to a unipolar operation of the electrode (3) and automatically applying the stored stimulation parameter values for a unipolar stimulation of the cardiac region.

## Patentansprüche

1. Implantierbare medizinische Vorrichtung zum Stimulieren eines menschlichen oder tierischen Herzens, umfassend ein Gehäuse (2), einen Prozessor, eine Speichereinheit, eine Stimulationseinheit, die zum Stimulieren einer Herzgegend eines menschlichen oder tierischen Herzens (5) konfiguriert ist, und eine Detektionseinheit, die zum Detektieren eines elektrischen Signals an der Herzgegend desselben Herzens (5) konfiguriert ist, wobei die Stimulationseinheit eine Elektrode (3) mit einem ersten Elektrodenpol (6) und einem zweiten Elektrodenpol (7) umfasst, wobei die Speichereinheit ein computerlesbares Programm umfasst, das, wenn es auf dem Prozessor ausgeführt wird, bewirkt, dass der Prozessor die folgenden Schritte ausführt:
a) Bestimmen geeigneter Stimulationsparameterwerte eines Stimulationspulses, der einer Herzgegend eines menschlichen oder tierischen Herzens (5) von dem ersten Elektrodenpol (6) auf unipolare Weise zugeführt werden soll;
b) Speichern der bestimmten Stimulationsparameterwerte in der Speichereinheit;
c) Überprüfen eines Auftretens eines Elektrodenausfalls der Elektrode (3), wenn die Elektrode (3) auf bipolare Weise betrieben wird;
d) im Fall des Auftretens eines Elektrodenausfalls automatisches Schalten von einem bipolaren Betrieb der Elektrode (3) zu einem unipolaren Betrieb der Elektrode (3) und automatisches Anwenden der gespeicherten Stimulationsparameterwerte für eine unipolare Stimulation der Herzgegend.

2. Implantierbare medizinische Vorrichtung nach Anspruch 1, **dadurch gekennzeichnet, dass** die Detektionseinheit dieselbe Elektrode (3) umfasst wie die Stimulationseinheit, wobei das computerlesbare Programm bewirkt, dass der Prozessor vor Schritt d) die folgenden Schritte ausführt: Bestimmen geeigneter Erfassungsparameterwerte zum Erfassen eines elektrischen Signals an der Herzgegend durch den ersten Elektrodenpol (6) auf unipolare Wiese und Speichern der bestimmten Erfassungsparameterwerte in der Speichereinheit, wobei das computerlesbare Programm bewirkt, dass der Prozessor in Schritt d) zusätzlich Folgendes ausführt: Anwenden der gespeicherten Erfassungsparameterwerte zum Erfassen eines elektrischen Signals an der Herzgegend.

3. Implantierbare medizinische Vorrichtung nach Anspruch 1 oder 2, **dadurch gekennzeichnet, dass** die Stimulationsparameter einen Wert einer Amplitude und/oder einen Wert einer Pulsbreite eines Stimulationspulses umfassen.

4. Implantierbare medizinische Vorrichtung nach einem der Ansprüche 1 bis 3, **dadurch gekennzeichnet, dass** die Herzgegend eine rechte Kammer des menschlichen oder tierischen Herzens (5) ist.

5. Implantierbare medizinische Vorrichtung nach einem der Ansprüche 1 bis 3, **dadurch gekennzeichnet, dass** die Herzgegend ein His-Bündel (4) des menschlichen oder tierischen Herzens (5) ist.

6. Implantierbare medizinische Vorrichtung nach einem der vorhergehenden Ansprüche, **dadurch gekennzeichnet, dass** die Schritte a), b), c) unabhängig voneinander automatisch oder auf Anforderung eines Benutzers ausgeführt werden.

7. Implantierbare medizinische Vorrichtung nach einem der vorhergehenden Ansprüche, **dadurch gekennzeichnet, dass** Schritt c) zyklisch wiederholt wird.

8. Implantierbare medizinische Vorrichtung nach einem der vorhergehenden Ansprüche, **dadurch gekennzeichnet, dass** Schritt c) durch Messen einer Impedanz (Z) zwischen dem ersten Elektrodenpol (6) und dem zweiten Elektrodenpol (7) und durch Bestimmen, ob die gemessene Impedanz (Z) niedriger als ein vorgegebener Schwellenwert der unteren Impedanz oder höher als ein vorgegebener Schwellenwert der oberen Impedanz ist, ausgeführt wird.

9. Anordnung, umfassend eine implantierbare medizinische Vorrichtung nach einem der vorhergehenden Ansprüche und eine Programmiervorrichtung zum Programmieren der implantierbaren medizinischen Vorrichtung (1).

10. Anordnung nach Anspruch 9, **dadurch gekennzeichnet, dass** die Programmiervorrichtung so konstruiert und angeordnet ist, dass sie ein Anpassen von Stimulationsparameterwerten eines Stimulationspulses, der einer Herzgegend eines menschlichen oder tierischen Herzens (5) von einem ersten Elektrodenpol (6) einer Elektrode (3) auf unipolare Weise zugeführt werden soll, ermöglicht und/oder das Speichern der Stimulationsparameterwerte ermöglicht.

11. Anordnung nach Anspruch 9 oder 10, **dadurch gekennzeichnet, dass** die Programmiervorrichtung so konstruiert und angeordnet ist, dass sie ein Anpassen von Erfassungsparameterwerten zum Erfassen eines elektrischen Signals an der Herzgegend eines menschlichen oder tierischen Herzens (5) von einem ersten Elektrodenpol (6) einer Elektrode (3) auf unipolare Weise ermöglicht und/oder das Speichern von Erfassungsparameterwerten ermöglicht.

12. Computerprogrammprodukt, das computerlesbaren Code umfasst, der bewirkt, dass die Vorrichtung nach Anspruch 1 die folgenden Schritte ausführt:
a) Bestimmen geeigneter Stimulationsparameterwerte eines Stimulationspulses, der einer Herzgegend eines menschlichen oder tierischen Herzens (5) von einem ersten Elektrodenpol (6) einer Elektrode (3) einer Stimulationseinheit einer implantierbaren medizinischen Vorrichtung (1) auf unipolare Weise zugeführt werden soll;
b) Speichern der bestimmten Stimulationsparameterwerte in einer Speichereinheit der implantierbaren medizinischen Vorrichtung (1);
c) Überprüfen eines Auftretens eines Elektrodenausfalls der Elektrode (3), wenn die Elektrode (3) auf bipolare Weise betrieben wird;
d) im Fall des Auftretens eines Elektrodenausfalls automatisches Schalten von einem bipolaren Betrieb der Elektrode (3) zu einem unipolaren Betrieb der Elektrode (3) und automatisches Anwenden der gespeicherten Stimulationsparameterwerte für eine unipolare Stimulation der Herzgegend.

## Revendications

1. Dispositif médical implantable destiné à stimuler un cœur humain ou animal, comprenant un boîtier (2), un processeur, une unité de mémoire, une unité de stimulation configurée pour stimuler une région cardiaque d'un cœur humain ou animal (5), et une unité de détection configurée pour détecter un signal électrique au niveau de la région cardiaque dudit cœur (5), dans lequel l'unité de stimulation comprend une électrode (3) ayant un premier pôle d'électrode (6) et un second pôle d'électrode (7), dans lequel l'unité de mémoire comprend un programme lisible par ordinateur qui amène le processeur à réaliser les étapes suivantes lorsqu'elles sont exécutées sur le processeur :
a) déterminer des valeurs de paramètres de stimulation appropriées d'une impulsion de stimulation à faire délivrer à une région cardiaque d'un cœur humain ou animal (5) par le premier pôle d'électrode (6) d'une manière unipolaire ;
b) stocker les valeurs de paramètres de stimulation déterminées dans l'unité de mémoire ;
c) vérifier une occurrence d'une défaillance de l'électrode (3) lors du fonctionnement de l'électrode (3) d'une manière bipolaire ;
d) dans le cas de l'occurrence d'une défaillance d'électrode, commuter automatiquement d'un fonctionnement bipolaire de l'électrode (3) à un fonctionnement unipolaire de l'électrode (3) et appliquer automatiquement les valeurs de paramètres de stimulation stockées pour une stimulation unipolaire de la région cardiaque.

2. Dispositif médical implantable selon la revendication 1, **caractérisé en ce que** l'unité de détection comprend la même électrode (3) en tant qu'unité de stimulation, dans lequel le programme lisible par ordinateur amène le processeur à réaliser les étapes suivantes avant l'étape d): déterminer les valeurs de paramètres de détection appropriées pour faire détecter un signal électrique au niveau de la région cardiaque par le premier pôle d'électrode (6) d'une manière unipolaire et stocker les valeurs de paramètres de détection déterminées dans l'unité de mémoire, dans lequel le programme lisible par ordinateur amène le processeur à réaliser de plus ce qui suit dans l'étape d) : appliquer les valeurs de paramètres de détection stockées pour détecter un signal électrique au niveau de la région cardiaque.

3. Dispositif médical implantable selon la revendication 1 ou 2, **caractérisé en ce que** le paramètre de stimulation comprend une valeur d'une amplitude et/ou une valeur d'une largeur d'impulsion d'une impulsion de stimulation.

4. Dispositif médical implantable selon l'une quelconque des revendications 1 à 3, **caractérisé en ce que** la région cardiaque est un ventricule droit du cœur humain ou animal (5).

5. Dispositif médical implantable selon l'une quelconque des revendications 1 à 3, **caractérisé en ce que** la région cardiaque est un faisceau de His (4) du cœur humain ou animal (5).

6. Dispositif médical implantable selon l'une quelconque des revendications précédentes, **caractérisé en ce que** les étapes a), b), c) sont, indépendamment les unes des autres, mises en œuvre automatiquement ou à la requête d'un utilisateur.

7. Dispositif médical implantable selon l'une quelconque des revendications précédentes, **caractérisé en ce que** l'étape c) est répétée de manière cyclique.

8. Dispositif médical implantable selon l'une quelconque des revendications précédentes, **caractérisé en ce que** l'étape c) est mise en œuvre en mesurant une impédance (Z) entre le premier pôle d'électrode (6) et le second pôle d'électrode (7) et en déterminant si l'impédance mesurée (Z) est inférieure à une valeur seuil d'impédance inférieure prédéterminée ou supérieure à une valeur seuil d'impédance supérieure prédéterminée.

9. Agencement comprenant un dispositif médical implantable selon l'une quelconque des revendications précédentes et un dispositif de programmation destiné à programmer le dispositif médical implantable (1).

10. Agencement selon la revendication 9, **caractérisé en ce que** le dispositif de programmation est conçu et agencé pour permettre un ajustement des valeurs de paramètres de stimulation d'une impulsion de stimulation à faire délivrer à une région cardiaque d'un cœur humain ou animal (5) par un premier pôle d'électrode (6) d'une électrode (3) d'une manière unipolaire et/ou pour permettre le stockage des valeurs de paramètres de stimulation.

11. Agencement selon la revendication 9 ou 10, **caractérisé en ce que** le dispositif de programmation est conçu et agencé pour permettre un ajustement des valeurs de paramètres de détection afin de faire détecter un signal électrique au niveau de la région cardiaque d'un cœur humain ou animal (5) par un premier pôle d'électrode (6) d'une électrode (3) d'une manière unipolaire et/ou pour permettre le stockage des valeurs de paramètres de détection.

12. Produit de programme informatique comprenant un code lisible par ordinateur qui amène le dispositif selon la revendication 1 à exécuter les étapes suivantes :
a) déterminer des valeurs de paramètres de stimulation appropriées d'une impulsion de stimulation à faire délivrer à une région cardiaque d'un cœur humain ou animal (5) par un premier pôle d'électrode (6) d'une électrode (3) d'une unité de stimulation d'un dispositif médical implantable (1) d'une manière unipolaire ;
b) stocker les valeurs de paramètres de stimulation déterminées dans une unité de mémoire du dispositif médical implantable (1) ;
c) vérifier une occurrence d'une défaillance de l'électrode (3) lors du fonctionnement de l'électrode (3) d'une manière bipolaire ;
d) dans le cas de l'occurrence d'une défaillance d'électrode, commuter automatiquement d'un fonctionnement bipolaire de l'électrode (3) à un fonctionnement unipolaire de l'électrode (3) et appliquer automatiquement les valeurs de paramètres de stimulation stockées pour une stimulation unipolaire de la région cardiaque.
